# FASCICULE DE BREVET EUROPEEN

(11) **EP 1 165 831 B1**
(45) Date de publication et mention de la délivrance du brevet: **31.05.2006**
(21) Numéro de dépôt: 00910913.3
(22) Date de dépôt: 09.03.2000
(51) Int. Cl.: C12Q 1/14

(54) **MILIEU CHROMOGENE DE DETECTION DE STAPHYLOCOCCUS AUREUS**
CHROMOGENES MEDIUM ZUM NACHWEIS VON STAPHYLOCOCCUS AUREUS
CHROMOGENIC MEDIUM FOR DETECTING STAPHYLOCOCCUS AUREUS

(30) Priorité: 11.03.1999 FR 9903003
(43) Date de publication de la demande: 02.01.2002
(73) Titulaire: Rambach, Alain, F-75006 Paris (FR)
(72) Inventeur: Rambach, Alain, F-75006 Paris (FR)
(74) Mandataire: Texier, Christian
(86) Numéro de dépôt international: PCT/FR2000/000583
(87) Numéro de publication internationale: WO 2000/053799

(56) Documents cités:
- WO-A-92/12259
- WO-A-95/04157
- WO-A-95/20674
- WO-A-98/32874
- FR-A- 2 747 394
- LINDSAY J A; ARAVENA-ROMAN M A; RILEY T V: "Identification of Staphylococcus epidermidis and Staphylococcus hominis from blood cultures by testing susceptibility to desferrioxamine" EUROPEAN JOURNAL OF CLINICAL MICROBIOLOGY & INFECTIOUS DISEASES, vol. 12, 1993, pages 127-131, XP000921267
- HEUCK DAGMAR; WITTE WOLFGANG; BRAULKE CHRISTINE; REISSBRODT ROLF: "Susceptibility to desferrioxamines and other chelators of coagulase-negative staphylococci" ZENTRALBLATT FUER BAKTERIOLOGIE, vol. 280, 1994, pages 304-311, XP000921273
- MULDER J G: "A simple and inexpensive method for the identification of Staphylococcus epidermidis and Staphylococcus hominis" EUROPEAN JOURNAL OF CLINICAL MICROBIOLOGY & INFECTIOUS DISEASES, vol. 14, 1995, pages 1052-1056, XP000921270
- D L STEVENS, C JONES: "Use of trehalose-mannitol-phosphatase agar to differentiate Staphylococcus aureus and Staphylococcus saprophyticus" JOURNAL OF CLINICAL MICROBIOLOGY, vol. 20, 1984, pages 977-980, XP000856420 cité dans la demande

## Description

La présente invention concerne un nouveau milieu chromogène destiné à mettre en évidence *Staphylococcus aureus.*

*Staphylococcus aureus* est une bactérie dont la détection se révèle de plus en plus intéressante car il s'agit de bactéries qui sont souvent portées par des patients devant subir des soins traumatiques, seringues, cathéter, ou opérations diverses. Dans ce cas, il existe un très grand danger d'infection à terme dès que ces patients entrent en soin.

Il s'agit donc d'une bactérie pathogène mais qui, de plus en plus, est impliquée dans des infections nocosomiales en milieux hospitaliers par exemple.

De fait, la surveillance épidémiologique des *Staphylococcus aureus* devient de plus en plus nécessaire et se généralise.

Le milieu classique de détection de *Staphylococcus aureus* dans le domaine clinique est un milieu mannitol-sel qui repose sur les caractéristiques de résistance au sel et d'acidification en présence du mannitol. Ce test n'est pas très satisfaisant, notamment pour ce qui concerne la sensibilité et la spécificité, ce milieu très sélectif conduit à un nombre trop élevé de faux négatifs et, par ailleurs, donne également des faux positifs. De plus, la coloration des colonies provenant de l'acidification diffuse dans le milieu, ce qui ne facilite pas la lecture lorsque l'échantillon n'est pas une culture pure et que les colonies isolées sont proches les unes des autres.

La technique antérieure a déjà suggéré l'utilisation de milieux alternatifs, notamment utilisant la phosphatase comme caractère de détection de *Staphylococcus aureus* (Stevens DL et Jones C: Use of trehalose-mannitol-phosphatase agar to differentiate *Staphylococcus aureus* and *Staphylococcus saprophyticus.* J. Clin. Microbiol. 20:977-980, 1984) avec par exemple un milieu contenant l'indicateur phénolphtaléine phosphate. Cependant, ce milieu présente le défaut de donner une coloration diffusible qui pose problème lorsque l'échantillon n'est pas une culture pure et, comme dans le cas précédent, lorsque les colonies isolées sont proches les unes des autres.

WO 95/20674 a pour objet une méthode de détection des staphylocoques comprenant l'inoculation, dans un milieu sélectif, d'un premier substrat capable de donner lieu à une première couleur en présence de β-glucosidase et un second substrat capable de produire une seconde couleur en présence de staphylocoques.

WO 98/32874 a pour objet un milieu de croissance pour la détection sélective des staphylocoques dans un milieu comprenant des *Bacillus,* ledit milieu comprenant notamment un glucopyranoside.

Heuck et al. (Zentralblatt fuer Bakteriologie, vol. 280, 1994, pages 304-311) concerne la mise en évidence de la susceptibilité de différents staphylocoques aux déféroxamines et autres chélateurs par la mise en oeuvre de tests d'inhibition réalisés au moyen de disques de papier filtre.

La présente invention repose sur l'utilisation d'un milieu chromogène contenant deux agents chromogènes permettant d'obtenir un milieu sensible ne donnant pratiquement pas de faux négatifs pour *S. aureus* et permettant, en outre, de différencier *S*. *aureus* des autres espèces telles que *Streptococcus.*

Lorsque l'on indique qu'un milieu "ne donne pas" ou "pratiquement pas" de faux positifs ou de faux négatifs, il faut toujours entendre que c'est par rapport aux souches qui ont été testées, il n'est pas possible d'être totalement affirmatif car des souches atypiques ou mutantes peuvent apparaître tous les jours.

Plus particulièrement, l'invention concerne un milieu de détection de *Staphylococcus aureus* contenant à titre-d'agent chromogène: du 5-bromo 6-chloro 3-indoxyl phosphate, dans un milieu de culture, et contenant en outre de la déféroxamine.

De préférence ledit milieu de culture comprendra en outre du 5-bromo-4-chloro-3-indoxyl flucoside. Lorsque l'on utilise le 5-bromo 6-chloro 3-indoxyl phosphate sans le dérivé 5-bromo 4-chloro 3-indoxyl glucoside, alors on utilisera de préférence une combinaison avec un substrat chromogène de la glucosidase, en général de type indoxyl glucoside.

Les milieux de culture de *S. aureus* sont connus et décrits notamment dans le manuel "Oxoïd Unipath Limited", Wade Road, Basingstoke, Hampshire, RG24 OPW, Angleterre. Il peut s'agir, par exemple de "Nutrient Agar Oxoïd CM3", milieu essentiellement à base d'extraits de levure, de peptone et d'agar.

Par "agent chromogène", on entend désigner un composé qui change de couleur en présence d'une souche déterminée, notamment sous l'action du système enzymatique de ladite souche.

L'utilisation du milieu selon l'invention permet d'observer une coloration mauve du 5-bromo 6-chloro 3-indoxyl phosphate en présence de *S. aureus* alors que le 5-bromo 4-chloro 3-indoxyl glucoside colore en bleu un grande nombre de souches de *Streptococcus* sans colorer *Staphylococcus aureus.*

Ceci est tout à fait inattendu car la littérature indique que *S. aureus* est β-glucosidase positive, comme de nombreuses souches de *Streptococcus,* ceci peut être vérifié en utilisant par exemple du para-nitrophényle β-glucoside. Or, on a fait l'observation surprenante que le 5-bromo 4-chloro 3-indoxyl glucoside colorait en bleu les souches de *Streptococcus* sans colorer les souches de *Staphylococcus aureus,* ceci a été vérifié sur un grande nombre de souches hospitalières de *S. aureus.*

Par ailleurs, la présence de la déféroxamine dans le milieu de culture selon la présente invention permet d'inhiber la croissance de *Staphylococcus epidermis* sans inhiber *Staphylococcus aureus,* ce qui permet de discriminer ces deux organismes. La concentration utilisée sera de préférence comprise entre 0,010 et 0,100 g/l.

Le milieu selon la présente invention peut également être amélioré en ajoutant l'un ou les deux chromogènes suivants :
- 5-bromo 4-chloro 3-indoxyl galactoside et
- 5-bromo 4-chloro 3-indoxyl glucuronide
qui sont deux caractères négatifs pour *Staphylococcus aureus* et permettent donc de le différencier des souches positives pour ces chromogènes.

Les milieux selon la présente invention contiendront, de préférence, de 0,01 à 0,500 g/l, notamment de 0,050 à 0,150 g/l de 5-bromo 6-chloro 3-indoxyl phosphate, de préférence de 0,010 à 0,200 g/l de 5-bromo 4-chloro 3-indoxyl glucoside, de préférence de 0,010 à 0,200 g/l de 5-bromo 4-chloro 3-indoxyl galactoside, de préférence de 0,010 à 0,200 g/l de 5-bromo 4-chloro 3-indoxyl glucuronide.

Un milieu préféré selon l'invention, qui permet de différencier les *Staphylococcus aureus* par la coloration mauve des colonies, tandis que d'autres souches sont inhibées ou donnent des colonies incolores ou bleues, est le suivant :
- Peptone et extrait de levure 50 g/l
- 5-bromo 6-chloro 3-indoxyl phosphate 0,100 gl
- 5-bromo 4-chloro 3-indoxyl glucoside 0,050 g/l
- 5-bromo 4-chloro 3-indoxyl galactoside 0,050 g/l
- 5-bromo 4-chloro 3-indoxyl glucuronide 0,050 g/l
- Déféroxamine 0,050 g/l
- Agar 15 g/l

D'autres caractéristiques et avantages de la présente invention apparaîtront à la lecture des exemples ci-après :

### EXEMPLES

Afin de mettre en évidence l'intérêt de l'utilisation de 5-bromo 6-chloro 3-indoxyl phosphate pour la mise en évidence de *S. aureus* on le compare ci-après avec un même type de chromogène phosphate, mais cette fois le 5-bromo 4-chloro 3-indoxyl phosphate.

Les résultats sont indiqués dans le tableau suivant:

| **Couleur des colonies après 24 heures à 37°C** | | |
|---|---|---|
| | **Milieu A** | **Milieu B** |
| *S. aureus* AR 3916 | bleu | mauve |
| *S. aureus* AR 3917 | incolore | mauve |
| *S. aureus* AR 3918 | bleu | mauve |

**Milieu A :** Nutrient Agar Oxoïd CM3 28 g/l,
   5-bromo 4-chloro 3-indoxyl phosphate 0,050 g/l
**Milieu B :** Nutrient Agar Oxoïd CM3 28 g/l,
   5-bromo 6-chloro 3-indoxyl phosphate 0,050 g/l

On constate que bien que les deux chromogènes soient excessivement voisins, le substrat 5-bromo 6-chloro 3-indoxyl phosphate permet d'écarter des faux négatifs sur la souche AR 3917 par rapport au dérivé correspondant 5-bromo 4-chloro.

## Revendications

1. Milieu de détection de *Staphylococcus aureus,* **caractérisé en ce qu'**il comporte dans un milieu de culture de *Staphylococcus aureus* du 5-bromo 6-chloro 3-indoxyl phosphate et qu'il contient en outre de la déféroxamine.

2. Milieu selon la revendication 1, **caractérisé en ce qu'**il contient en outre du 5-bromo-4-chloro-3-indoxyl glucoside.

3. Milieu selon l'une des revendications 1 et 2, **caractérisé en ce qu'**il comporte, en outre, au moins l'un des deux agents chromogènes suivants :
- 5-bromo 4-chloro 3-indoxyl galactoside et
- 5-bromo 4-chloro 3-indoxyl glucuronide.

4. Milieu selon l'une des revendications 1 à 3, **caractérisé en ce qu'**il contient :
- Peptone et extrait de levure 50 g/l
- 5-bromo 6-chloro 3-indoxyl phosphate 0,100 gl
- 5-bromo 4-chloro 3-indoxyl glucoside 0,050 g/l
- 5-bromo 4-chloro 3-indoxyl galactoside 0,050 g/l
- 5-bromo 4-chloro 3-indoxyl glucuronide 0,050 g/l
- Déféroxamine 0,050 g/l
- Agar 15 g/l

## Claims

1. Medium for the detection of *Staphylococcus aureus,* **characterized in that** it comprises, in a *Staphylococcus aureus* culture medium, 5-bromo-6-chloro-3-indoxyl phosphate and **in that** it additionally comprises deferoxamine.

2. Medium according to Claim 1, **characterized in that** it additionally comprises, 5-bromo-4-chloro-3-indoxyl glucoside.

3. Medium according to either of Claims 1 and 2, **characterized in that** it additionally comprises at least one of the following two chromogenic agents:
- 5-bromo-4-chloro-3-indoxyl galactoside and
- 5-bromo-4-chloro-3-indoxyl glucuronide.

4. Medium according to one of Claims 1 to 3, **characterized in that** it comprises:
- Peptone and yeast extract 50 g/l
- 5-bromo-6-chloro-3-indoxyl phosphate 0.100 g/l
- 5-bromo-4-chloro-3-indoxyl glucoside 0.050 g/l
- 5-bromo-4-chloro-3-indoxyl galactoside 0.050 g/l
- 5-bromo-4-chloro-3-indoxyl glucuronide 0.050 g/l
- Deferoxamine 0.050 g/l
- Agar 15 g/l

## Patentansprüche

1. Medium für den Nachweis von *Staphylococcus aureus,* **dadurch gekennzeichnet, dass** es in einem Kulturmedium für *Staphylococcus aureus* 5-Brom-6-chlor-3-indoxylphosphat umfasst und dass es außerdem Deferoxamin enthält.

2. Medium nach Anspruch 1, **dadurch gekennzeichnet, dass** es außerdem 5-Brom-4-chlor-3-indoxylglucosid enthält.

3. Medium nach einem der Ansprüche 1 und 2, **dadurch gekennzeichnet, dass** es außerdem wenigstens eines der beiden folgenden chromogenen Mittel umfasst:
- 5-Brom-4-chlor-3-indoxylgalactosid und
- 5-Brom-4-chlor-3-indoxylglucuronid.

4. Medium nach einem der Ansprüche 1 bis 3, **dadurch gekennzeichnet, dass** es enthält:
- Pepton und Hefeextrakt 50 g/l
- 5-Brom-6-chlor-3-indoxylphosphat 0,100 g/l
- 5-Brom-4-chlor-3-indoxylglucosid 0,050 g/l
- 5-Brom-4-chlor-3-indoxylgalactosid 0,050 g/l
- 5-Brom-4-chlor-3-indoxylglucuronid 0,050 g/l
- Deferoxamin 0,050 g/l
- Agar 15 g/l.
